Europäisches Patentamt

European Patent Office

Office européen des brevets

(11) Numéro de publication: **0 116 240**
**A1**

## (12) DEMANDE DE BREVET EUROPEEN

(21) Numéro de dépôt: **83401388.0**

(22) Date de dépôt: **06.07.83**

(51) Int. Cl.³: **A 61 L 15/03**
**//A61K35/02**

(30) Priorité: **07.01.83 FR 8300201**

(43) Date de publication de la demande:
**22.08.84 Bulletin 84/34**

(84) Etats contractants désignés:
**BE DE GB IT**

(71) Demandeur: **Heitz, Jean**
**203, Allée de Montfermeil**
**F-93390 Clichy-Sous-Bois(FR)**

(72) Inventeur: **Heitz, Jean**
**203, Allée de Montfermeil**
**F-93390 Clichy-Sous-Bois(FR)**

(74) Mandataire: **Laget, Jean-Loup et al,**
**Cabinet Pierre Loyer 18, Rue de Mogador**
**F-75009 Paris(FR)**

(54) **Bande à charge active formée d'argile verte et procédé pour sa fabrication.**

(57) Cette bande, qui est utilisable notamment à des fins de traitement thérapeutique pour l'homme et les animaux, comprend un support à structure aérée portant une charge d'argile verte adhérant sur lui. Cette bande peut être présentée en grandes longueurs, en étant alors enroulée autour d'un noyau. Il suffit, pour son utilisation, de dérouler la longueur de bande requise et de l'appliquer sur la partie à traiter. Sa fabrication est effectuée en préparant une suspension d'argile verte dans un milieu liquide formé par une colle et un solvant ou dispersant pour celle-ci, pour constituer une masse pâteuse ou visqueuse qui est appliquée sur le support par enduction. Le solvant ou dispersant est de nature organique et la colle est avantageusement un composé cellulosique tel qu'un éther de cellulose.

## Bande à charge active formée d'argile verte et procédé pour sa fabrication

L'emploi de l'argile à des fins médicales est connu depuis très longtemps, et les propriétés thérapeutiques de certaines argiles ont été étudiées depuis de nombreuses années.

Parmi les argiles, les propriétés de l'argile verte en particulier, ont retenu l'attention des chercheurs.

L'argile verte thérapeutique a en effet des propriétés générales de décongestion, de désinfection, de cicatrisation et d'apaisement des douleurs.

Ainsi, l'argile verte thérapeutique referme les plaies sans irritations locales et sans proliférations microbiennes, ce qui élimine les risques d'inflammation, en faisant disparaître l'humeur. Elle sèche les brûlures et referme les plaies ulcéreuses. Elle améliore la circulation du sang et de ce fait fait disparaître les veinules et les varices. Elle agit également ment dans les cas d'arthrose et calme la douleur.

On sait que l'argile verte thérapeutique renferme les constituants suivants : silice, oxyde de fer, oxyde d'aluminium, manganèse, oxyde de zinc, magnésie, potassium, chaux, soude, chlore, sulfates, phosphore, carbonates, or, argent et calcium.

Parmi ces constituants, le phosphore, en équilibre avec le calcium, intervient dans l'édification osseuse. Le magnésium intervient dans de nombreuses réactions vitales ; il assure les liaisons riches en énergie des glucides et possède une activité enzymatique. Le calcium contribue à l'équilibre ionique du sang. Le potassium est nécessaire aux échanges intracellulaires à travers les membranes et à la libération de l'énergie musculaire.

On a déjà proposé d'utiliser l'argile verte notamment en bains, en applications, etc. ...

L'invention concerne un produit permettant une exploitation aisée et commode des propriétés de l'argile verte pour le traitement localisé de plaies, douleurs, etc... aussi bien chez l'homme que chez les animaux.

L'invention est matérialisée, à titre de produit nouveau, dans une bande utilisable notamment à des fins de traitement thérapeutique chez l'homme et les animaux, comprenant un support à structure aérée portant une charge d'argile verte adhérant sur lui.

Il en résulte l'obtention d'un produit particulièrement simple à utiliser, puisqu'il suffit à la personne qui l'emploie d'appliquer cette bande sur la partie à traiter, qu'il s'agisse du corps humain ou du corps d'un animal, l'application pouvant s'effectuer soit par enveloppement, soit par simple recouvrement, de la même manière que dans le cas d'un emplâtre connu.

Le support du produit objet de l'invention est judicieusement aéré pour permettre la pénétration de l'argile verte dans toute l'épaisseur du support et ainsi un meilleur accrochage entre l'argile et ce support, de même que l'obtention d'une bande garnie d'une couche d'argile verte sur ses deux faces.

Ce support aéré peut être en fibres naturelles ou synthétiques mais un support en coton aéré (gaze) semble convenir de façon particulièrement bonne.

Le produit faisant l'objet de l'invention peut être sous la forme de bandes relativement courtes, pouvant être appliquées sur la partie du corps à traiter, mais il se présente avantageusement sous la forme d'une bande de longueur notable enroulée sur elle-même, de préférence autour d'un noyau central, par exemple en matière plastique. La bande peut être notamment livrée au commerce en longueurs de 3 ou 5 mètres par exemple, ou plus. Il suffit alors à l'utilisateur de dérouler la longueur de bande appropriée pour l'application sur la partie à traiter ou son enveloppement, la bande pouvant dans chaque cas être disposée en une ou plusieurs couches ou épaisseurs sur la partie à traiter. Il est possible de réaliser des bandes de largeurs différentes, par exemple de 5cm, 8cm, 12cm, etc..., selon l'utilisation envisagée.

Les recherches effectuées ont permis de constater qu'on obtient des résultats de traitement très satisfaisants, dans la plupart des cas, par l'application sur la partie à traiter d'une longueur de bande appropriée imbibée d'eau, en laissant la bande en place pendant une ou deux heures, suivant le traitement.

Si désiré, d'autres produits ou agents de traitement peuvent être incorporés à la bande pour obtenir par exemple un effet thérapeutique spécifique.

Mais le produit faisant l'objet de l'invention peut également être utilisé pour le traitement de végétaux, par exemple par enroulement autour d'un arbre. On a constaté en effet que, dans ce cas encore, l'argile verte formant la charge active de la bande a des effets de traitement très favorables sur le végétal.

Pour une telle application, la composition de la bande peut être complétée par l'incorporation à l'argile verte de certains constituants, comme des essences naturelles, qui agissent sur les végétaux pour en favoriser la croissance tout en s'opposant à une attaque par certains insectes nuisibles.

L'invention concerne encore un procédé pour la fabrication de la bande à charge active formée d'argile verte thérapeutique telle que spécifiée ci-avant.

Un problème qui se pose pour la réalisation d'une telle bande chargée d'argile réside dans le moyen permettant de faire adhérer l'argile de façon suffisante et définitive sur le support. En effet, lorsque l'argile verte, qui est initialement à l'état de matière pulvérulente, est mouillée pour former une masse pâteuse pouvant être appliquée sur un support, puis séchée après son application, elle perd ses propriétés et a en outre tendance à se détacher du support.

Le procédé faisant l'objet       de l'invention consiste à préparer une suspension d'argile verte dans un milieu liquide formé par une colle et un agent solvant ou dispersant pour celle-ci, afin de constituer une masse pâteuse ou assez visqueuse à appliquer cette masse  sur le support, notamment par enduction, puis à sécher l'ensemble pour provoquer l'élimination de l'agent solvant ou dispersant, ce qui laisse le produit constitué par une bande de support sur laquelle l'argile verte adhére  de façon parfaite.

La préparation de la suspension d'argile verte destinée à la mise en oeuvre du procédé peut s'effectuer de manière extrèmement simple, par introduction de l'argile verte à l'état de poudre dans un liquide constitué par l'agent solvant ou dispersant et la colle elle-même.

Suivant l'invention, on peut utiliser un solvant ou agent dispersant de nature organique, tel que du chlorure de méthylène par exemple. La colle incorporée à ce solvant ou dispersant est judicieusement un produit cellulosique tel qu'un éther de cellulose.

Comme indiqué précédemment, on prépare à partir des constituants précités une masse pâteuse ou visqueuse, qui est appliquée sur le support par exemple en un poste d'enduction à la racle, d'une manière en soi classique pour des opérations d'enduction. Le support constitué de préférence par une bande de grande largeur défile au poste d'enduction et, du fait de la nature aérée de ce support, la masse pâteuse pénètre intimement dans sa structure, en réalisant un parfait accrochage.

A la sortie du poste d'enduction, le produit formé est acheminé à travers un tunnel de séchage, ce qui provoque l'élimination de l'agent solvant ou dispersant. La température régnant dans le tunnel doit être assez basse pour ne pas nuire à la constitution et à l'aspect final du produit, résultat qui peut être obtenu par un choix judicieux de l'agent solvant ou dispersant. Par exemple, une température de l'ordre de 40°C peut être utilisée dans le cas de chlorure de méthylène. Il est également possible, si désiré, de réaliser le séchage du produit à l'air, mais on conçoit aisément que l'utilisation d'un tunnel de séchage permet d'accélerer la fabrication.

Après séchage, la bande produite est coupée à la largeur requise et peut être enroulée autour d'un noyau, par exemple d'un noyau en matière plastique, puis coupée à la longueur désirée, ou bien elle peut être découpée directement en bandes courtes maintenues à plat. Comme indiqué précédemment, l'utilisateur pourra alors prélever à un rouleau de bande la longueur requise pour l'application ou le traitement envisagé.

L'invention concerne encore les bandes à charge active d'argile verte fabriquées par le procédé décrit.

On donnera ci-après un exemple de mise en oeuvre permettant de mieux comprendre le procédé suivant l'invention.

Pour la production de la bande à charge active d'argile faisant l'objet de l'invention, on part d'une bande de coton aéré (gaze) de grande largeur, par exemple d'une largeur de l'ordre d'un mètre. On prépare une charge pâteuse, à partir d'argile verte thérapeutique à l'état de poudre, que l'on introduit dans un liquide constitué par un solvant formé de chlorure de méthylène et une colle constituée par un éther cellulosique.
On utilise, pour la production de cette charge pâteuse, une
quantité de solvant représentant de 50 à 60 litres pour 100kg
d'argile, et une quantité de colle correspondant à 2kg environ
pour 100kg d'argile.

La charge pâteuse ainsi préparée est ensuite appliquée sur la
bande de coton par enduction, à raison de 400 à 600g d'argile
au mètre carré. Compte tenu de la nature aérée de la bande de
coton , la masse pâteuse pénètre intimement dans cette bande
et forme une couche sur ses deux faces. L'enduction est effectuée judicieusement à la racle, par un processus en continu.

La bande munie de sa charge de masse pâteuse est alors acheminée à travers un tunnel de séchage chauffé à une température
de l'ordre de 40°C. Après séchage, la bande de grande largeur
est découpée en bandes de 10 à 12 cm de large par exemple, destinée  à être livrée au commerce. Les bandes ainsi obtenues
sont également coupées en longueurs de 5 mètres environ et enroulées sur un noyau en matière plastique.

## Revendications de brevet

1. A titre de produit nouveau, bande à charge active utilisable notamment à des fins de traitement thérapeutique chez l'homme et les animaux, caractérisée en ce qu'elle comprend un support à structure aérée portant une charge d'argile verte adhérant sur lui.

2. Bande suivant la revendication 1, caractérisée en ce que la charge d'argile verte est présente sur les deux faces du support et imprègne intimement celui-ci.

3. Bande suivant la revendication 1 ou 2, caractérisée en ce que le support aéré est en fibres naturelles, notamment en coton.

4. Bande suivant la revendication 1 ou 2, caractérisée en ce que le support est en fibres synthétiques.

5. Bande suivant l'une quelconque des revendications précédentes, caractérisée en ce qu'elle se présente sous la forme d'une bande de grande longueur enroulée autour d'un noyau et pouvant être déroulée et coupée à la longueur requise pour son application.

6. Bande suivant l'une quelconque des revendications 1 à 4, caractérisée en ce qu'elle se présente sous forme de bande courte demeurant à plat.

7. Bande suivant l'une quelconque des revendications précédentes, caractérisée en ce qu'elle est imprégnée d'essences naturelles ou d'un autre produit de traitement.

8. Procédé pour la fabrication d'une bande à charge active formée d'argile verte, utilisable notamment à des fins de traitement thérapeutique chez l'homme et les animaux, caractérisé en ce qu'on préparé une suspension d'argile verte dans un milieu liquide formé par une colle et un agent solvant ou dispersant pour celle-ci, afin de constituer une masse pâteuse ou visqueuse, on applique cette masse formée par l'agent solvant ou dispersant, la colle et l'argile verte sur le support, notamment par enduction, puis on sèche l'ensemble pour éliminer l'agent solvant ou dispersant, ce qui laisse le produit constitué par une bande de support sur laquelle adhère l'argile verte.

9. Procédé suivant la revendication 8, caractérisé en ce qu'on prépare la suspension par introduction d'argile verte à l'état pulvérulent dans le liquide formé par l'agent solvant ou dispersant et la colle.

10. Procédé suivant la revendication 8 ou 9, caractérisé en ce qu'on utilise un agent solvant ou dispersant de nature organique.

11. Procédé suivant la revendication 10, caractérisé en ce que cet agent solvant est du chlorure de méthylène.

12. Procédé suivant l'une quelconque des revendications 8 à 11, caractérisé en ce que la colle est un produit cellulosique, notamment un éther de cellulose.

13. Procédé suivant l'une quelconque des revendications 8 à 12, caractérisé en ce qu'on sèche le produit résultant de l'opéraration d'enduction par passage dans un tunnel de séchage chauffé.

14. Bande à charge active d'argile verte utilisable notamment à des fins de traitement thérapeutique chez l'homme et les animaux, produite par le procédé suivant l'une quelconque des revendications 8 à 13.

Office européen
des brevets

**RAPPORT DE RECHERCHE EUROPEENNE**

EP  83 40 1388

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | GB-A-1 155 440  (H.F. KAMP)<br><br>*  Page  7, lignes 34-44; page 8, lignes  1-20; page 13, lignes 7-64; revendications 1,16,17 * | 1-6,8 10,13, 14 | A 61 L  15/03  //<br>A 61 K  35/02 |
| Y | | 7,12 | |
| | --- | | |
| X | US-A-1 504 911  (A. SCHINDLER-JENNY)<br><br>* En entier * | 1-6,8 10,13, 14 | |
| Y | | 7,12 | |
| | --- | | **DOMAINES TECHNIQUES RECHERCHES (Int. Cl. ³)** |
| X | THE MERCK INDEX, 9ième édition, 1976, ed. M. Windholz, pub. Merck & Co., Inc., Rahway, N.J., USA<br>* Page 693, no. 5133: "Kaolin" * | 1 | A 61 L  15/03 |
| | --- | | |
| X | DE-C-  231 495  (M. KOHN)<br><br>* En entier * | 1-6,8 10,13, 14 | |
| Y | | 7,12 | |
| | ---          -/- | | |

Le présent rapport de recherche a été établi pour toutes les revendications

| Lieu de la recherche<br>LA HAYE | Date d'achèvement de la recherche<br>11-04-1984 | Examinateur<br>PELTRE CHR. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

\& : membre de la même famille, document correspondant

OEB Form 1503 03 82

Office européen
des brevets

**RAPPORT DE RECHERCHE EUROPEENNE**

**0116240**
Numéro de la demande

EP  83 40 1388

| | **DOCUMENTS CONSIDERES COMME PERTINENTS** | | Page 2 |
|---|---|---|---|
| Catégorie | Citation du document avec indication, en cas de besoin, des parties pertinentes | Revendication concernée | CLASSEMENT DE LA DEMANDE (Int. Cl. 3) |
| Y | FR-A-2 206 099  (TEIJIN)<br>* Page 5, lignes 37-39; page 6, lignes 1-22 *<br><br>--- | 7,12 | |
| A | FR-A-2 489 689  (J. HEITZ)<br>* Page 2, lignes 6-30; revendication 7 *<br><br>--- | 1 | |
| A | FR-A-2 096 036  (A. BEAUFOUR)<br><br>--- | | |
| A | US-A-2 790 944  (D.H. TOLLSTRUP)<br><br>--- | | |
| A | US-A-3 935 363  (N.D. BURKHOLDER et al.)<br><br>----- | | DOMAINES TECHNIQUES RECHERCHES (Int. Cl. 3) |

Le présent rapport de recherche a été établi pour toutes les revendications

| Lieu de la recherche<br>LA HAYE | Date d'achèvement de la recherche<br>11-04-1984 | Examinateur<br>PELTRE CHR. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82